# EUROPEAN PATENT APPLICATION

(11) **EP 2 604 288 A1**
(43) Date of publication of application: **19.06.2013**
(21) Application number: 11398008.0
(22) Date of filing: 16.12.2011
(51) Int. Cl.: A61K 49/00, A61K 49/18, A61K 47/48

(54) **Nanoparticles and uses thereof**

(71) Applicant: Biocant - Associação De Transferência De Tecnologia, Cantanhede 3060-197 (PT)
(72) Inventor: Da Silva Ferrreira, Lino, 3030-076 Coimbra (PT); Neves Pires das Neves, Ricardo, 3060-197 Cantanhede (PT); Mota Gomes, Renata Sofia, 3060-197 Cantanhede (PT)
(74) Representative: Sampson, Eimear

(57) **Abstract**

*A nanoparticle conjugate for intracellular delivery of a therapeutic agent, wherein the conjugate comprises a nanoparticle, a cationic agent and the therapeutic agent.*

## Description

### Field of the Invention

The invention relates to nanoparticles (NPs) for intracellular delivery of therapeutic agents. In particular, the invention relates to a NP conjugate comprising a NP, a cationic agent and a therapeutic agent.

### Background of the Invention

NPs are very promising for the intracellular delivery of anticancer and immunomodulatory drugs, stem cell differentiation biomolecules and cell activity modulators. They can be designed to target specific organs and to release their payload at specific sub-cellular regions with desired kinetics (Ferreira L et al. (2008) Cell Stem Cell 3: 136-146). Although initial studies in the area of intracellular drug delivery have been performed in the delivery of deoxyribonucleic acid (DNA), there is an increasing interest in the use of other molecules to modulate cell activity.

The requirements on drug delivery systems are generally to improve efficacy and to reduce toxicity through enhancement of specificity. The application of NPs in intracellular drug delivery is advantageous over traditional delivery systems, as they have a high drug loading capacity, are stable in aqueous environments and for long-term storage, are non-toxic and non-immunogenic and may be targeted *in vivo* using antibodies or other ligands, for example.

Furthermore, these NPs can encapsulate or be conjugated to probes so that they can be visualised non-invasively by magnetic resonance imaging (MRI), fluorescence imaging and such like. Accordingly, they could be useful in measuring, understanding and manipulating cells, both *in vitro* and *in vivo.* Monitoring *in vivo* cell trafficking by non-invasive means is also critical to improving cellular therapeutics, drug delivery and understanding disease progression.

However, pharmacokinetics and cell penetration represent potential obstacles to therapeutic formulations having an intracellular component. For example, the dose and bioactivity of therapeutic agents released by NPs is dependent on several variables including (i) the kinetics of cellular attachment and internalisation, (ii) the intracellular pathway and final location of the NPs and (iii) the cell cycle. The kinetics of cellular attachment and internalisation is affected by NP size, shape, charge and surface chemistry/bioactivity (Ferreira L (2009) J Cell Biochem 108: 746-752; Sahay G et al. (2010) J Control Release 145: 182-195; Duncan (2011) Curr Opin Biotechnol 22(4): 492-501). The mitotic index affects also the concentration of the drug that is released over time. The partitioning of NPs present in the cell during cell division is random and asymmetric and, thus, the concentration of NPs in each daughter cell after division may be different (Summers HD et al. (2011) Nat Nanotechno/ 6: 170-174). Finally, the route of NP internalisation and its final location within the cell plays an important role in the delivered dose and its bioactivity. The cell has a high number of compartments and, therefore, it is important that the NP releases its content at the right place in order to have the desired magnitude and duration of effect.

So far, there is no formulation for the *in vivo* delivery of therapeutically active agents, with simultaneous control in tracking, pharmacokinetics and internalisation.

### Summary of the Invention

In a first aspect, the invention provides a nanoparticle conjugate for intracellular delivery of a therapeutic agent, wherein the conjugate comprises a nanoparticle, a cationic agent and the therapeutic agent.

In an embodiment, the cation agent is a polycationic agent.

In an embodiment, the polycationic agent is selected from the group consisting of chitosan, peptides, peptide derivatives, polyethylenimine, poly(amido ethylenimine), poly(amido)amines, poly(disulphide)amines and protamine sulphate. Preferably, the polycationic agent is protamine sulphate.

In an embodiment, the intracellular delivery is mediated by endosomes. Preferably, the nanoparticles are retained in the endolysosomal compartment.

In an embodiment, the nanoparticle is polymeric, preferably formed from poly(lactic acid-co-glycolic acid).

In an embodiment, the conjugate further comprises at least one imaging agent. The at least one imaging agent is preferably selected from the group consisting of perfluorocarbons and fluorescent labels. The perfluorocarbon is preferably perfluoro-1,5-crown ether.

In an embodiment, the therapeutic agent is a protein, peptide or oligonucleotide. Preferably, the oligonucleotide is miRNA.

A method of preparing a nanoparticle conjugate according to the invention in its first aspect is also envisaged.

Also provided is a NP conjugate comprising a NP and a cationic agent. The conjugate further comprises an imaging agent and/or a therapeutic agent. In an embodiment, the conjugate comprises an imaging agent and thus can be used for cell imaging, such as cell tracking. In an alternative embodiment, the conjugate comprises a therapeutic agent and can be used for intracellular delivery of said agent. In a preferred embodiment, the conjugate comprises an imaging agent and a therapeutic agent and thus can be used for simultaneous imaging and drug delivery.

### Brief Description of the Drawings

So that the present invention may be more fully understood, embodiments thereof will now be described with reference to the accompanying drawings in which:
Figure 1 illustrates a schematic representation of the preparation of a NP conjugate;
Figure 2 illustrates transmission electron microscopy (TEM) of NPs;
Figure 3 illustrates mass loss of poly(lactic acid-co-glycolic acid)-perfluoro-1,5-crown ether (PLGA-PPFCE) NPs over time;
Figure 4 illustrates the diameter of PLGA-PFCE NPs over time;
Figure 5 illustrates ¹⁹F nuclear magnetic resonance (NMR) spectrum of PLGA-PFCE NPs;
Figure 6A illustrates confocal laser scanning microscopy showing uptake of PLGA-PFCE formulation by HUVECs;
Figure 6B illustrates confocal laser scanning microscopy showing uptake of PLGA-PFCE formulation by mononuclear cells (MNCs);
Figure 7 illustrates MRI of PLGA-PFCE NPs (8 mg), PFCE (750 µl), PLGA-PFCE-PS-labelled human umbilical vascular endothelial cells(HUVECs) (10 x10⁶ cells) and unlabelled HUVECs (10 x 10⁶ cells) contained in separate eppendorfs;
Figure 8 illustrates fluorescence activated cell sorting (FACS) analysis of cell viability;
Figure 9 illustrates cell survival as a function of protamine sulphate concentration;
Figure 10 illustrates FACS analysis of uptake of fluorescently labelled PLGA-PFCE by HUVECs and MNCs in the presence or absence of a variety of inhibitors;
Figure 11 illustrates FACS analysis of cell NP labelling;
Figure 12 illustrates confocal laser scanning microscopy showing uptake of PLGA-PFCE-micro-ribonucleic acid (miRNA) formulation by HUVECs;
Figure 13 illustrates pro-survival activity of miRNA formulations;
Figure 14 illustrates vascular studies on cells transfected with PLGA-PFCE-miRNA (tube length); and
Figure 15 illustrates vascular studies on cells transfected with PLGA-PFCE-miRNA (branching points).

### Detailed Description of the Invention

The invention is directed to a novel type of NP that can be used to deliver therapeutic agents into cells. In a preferred embodiment, the NPs can be used simultaneously for cell tracking and drug delivery. The NPs can be rapidly internalised by mammalian cells, including endothelial, mononuclear and cardiac progenitor cells, where they are believed to largely remain at the endosomal compartment over time.

The term "nanoparticle" as used herein is taken to mean particles having one or more dimensions at the nanometre scale, i.e. those particles having one or more dimensions of from about 1 nm to about 1000 nm. Thus, the term "nanoparticle" encompasses both ultrafine particles (of from about 1 nm to about 100 nm) and fine particles (of from about 100 nm to about 500 nm).

### 1. Preparation of NPs

Examples of NPs include polymeric NPs, liposomes, carbon nanomaterials, ceramic NPs, emulsions, metallic particles, quantum dots and others (Ferreira L et al. (2008) Cell Stem Cell 3: 136-146). The NPs may be customised in terms of size, surface charge and attachment of any targeting moieties or the like. Any type of NP may be used in the present invention; preferably, however, the NPs are polymeric NPs.

The NPs of the present invention may be formed from any suitable biocompatible materials, which may be biodegradable or non-biodegradable. Examples of suitable biodegradable materials include collagen, fibrin, chitosan, hyaluronic acid, dextran, poly(anhydrides), poly(hydroxy acids), poly(ortho esters), poly(propylfumerates), poly(caprolactones), polyamides, polyamino acids, polyacetals, biodegradable polycyanoacrylates, biodegradable polyurethanes, poly(glycerol sebacates), especially elastomeric poly(glycerol sebacates), and polysaccharides. Non-biodegradable, yet biocompatible, materials include polypyrrole, polyanilines, polythiophene, polystyrene, polyesters, non-biodegradable polyurethanes, polyureas, poly(ethylene vinyl acetate), polypropylene, polymethacrylate, polyethylene, polycarbonates, and poly(ethylene oxide). Those skilled in the art will recognise that this is not a comprehensive list of materials appropriate for the preparation of NPs, but rather an illustrative list.

In a particularly preferred embodiment, the NPs are formed from poly(lactic acid-co-glycolic acid) (PLGA). PLGA is particularly suitable for use in the present invention because it is FDA-approved for human use, easily degraded and metabolised *in vivo* and formulations comprising PLGA are stable in aqueous environments and for long-term storage.

A suitable method for preparing NPs from PLGA is by using a spontaneous emulsification solvent diffusion technique (a so-called "single emulsion" (SE)), in which a single-phase co-solvent system in which the polymer (PLGA), and any molecules to be encapsulated, are soluble. After preparation the NPs may be centrifuged and washed before freeze-drying.

Alternatively NPs may be prepared using a double emulsion (DE) technique. This method requires the components to be mixed by sonication or homogenisation (Tabata et al. (1993) Pharm Res 10: 487-496; van de Weert et al. (2000) Pharm Res 17: 1159-1167). Typically, the DE method gives rise to NPs with a more homogeneous size distribution than the SE method. However, the SE method is simpler and more convenient than the DE method.

The size and morphology of the NPs can be controlled by factors such as the molecular weight of the polymer, polymer concentration, PVA concentration, organic solvent used to solubilise PLGA, aqueous phase pH and aqueous to organic phase (w/o) volume ratio (Song et al. (2008) Eur J Pharm Biopharm 69: 445-453; Feczkó et al. (2011) Chem Eng Process 50: 846-853; Mundargi et al. (2008) J Control Release 125: 193-209).

In alternative embodiments, the NPs can be prepared by the complexation of a polycation with a polyanion. In one embodiment, NPs are formed by the complexation of polyethylenimine (a polycation) and dextran sulfate (a polyanion) *(*Maia et al. (2011) ACS Nano 5(1): 97-106). In another embodiment, the polycations are reducible cationic polymers composed of low-molecular weight polycations crosslinked with disulphide linkages including poly(amido ethylenimine) (Jeong et al. (2007) Biomaterials 28: 1912-1917), poly(amido amine)s (Lin et al. (2007) Bioconjugate Chem 18: 138-145) and poly(disulphide amine)s (Ou et al. (2009) Biomaterials 30: 5804-5814). In another embodiment, the polyanion is hyaluronic acid.

The foregoing methods may be adapted depending upon the materials used to make the NPs, which adaptation will be within the remit of the skilled person.

### 2. Inclusion of an Imaging Agent

In a preferred embodiment, the NPs include an imaging agent. As used herein, the term "imaging agent" can mean any agent that can be tracked non-invasively using MRI, ultrasound, fluorescence imaging, confocal microscopy or such like. Suitable imaging agents include, for example fluorine compounds, such as perfluorocarbon moieties (PFCs), and fluorescent labels, such as fluorescent dyes.

PFCs have been in clinical use for decades and, as such, their biocompatibility is proven. However, PFCs are largely insoluble and potentially toxic in aqueous environments. Emulsification with lipid surfactants is one known solution to this problem, but the emulsions so produced tend to be unstable and it is challenging to attach active agents, targeting moieties or such like to an emulsion. The NPs described herein are thus advantageous, as they can avoid the need to form an emulsion, yet enable a substantial amount of PFCs to be carried. The NPs may be customised in terms of the amount and type of PFC included.

Suitable PFCs for use in the present invention include perfluorooctylbromide, perfluorodecalin, perfluorohexane, perfluoro-1,8-dicholorooctane, tris(trifluoromethyl)benzene and such like. Preferably, however, perfluoro-1,5-crown ether (PFCE) is used, which can be tracked non-invasively using ¹⁹F-MRI. NPs can encapsulate different PFCs having different MRI signatures. Therefore, a variety of PFCs can be used in order to allow more detailed information to be obtained.

Alternatively, or additionally, the NPs may include a fluorescent label, such as a fluorescent dye. These can be tracked non-invasively using fluorescence imaging, confocal microscopy or such like. Examples of suitable fluorescent labels include fluorescein (such as fluoresceinimine or fluorescein isothiocyanate (FITC)), rhodamine, Alexa Fluor® dyes, DyLight® Fluor dyes, ATTO dyes, boron-dipyrromethene (BODIPY) dyes and such like.

It is preferred that the NPs include at least one imaging agent, as this permits the NPs to be tracked in cells *in vitro* and/or *in vivo.* For example, they may include a PFC, but no fluorescent label, or *vice versa.* Alternatively, and preferably, they may include both an imaging agent such as an ultrasound imaging agent and a fluorescent label.

The imaging agent may be included in the NPs by any suitable means including encapsulation, covalent conjugation, physical immobilisation (for example, by electrostatic attraction, hydrophobic interaction and such like), layer-by-layer (LBL) adsorption and so on. The particular method used will depend upon the particular imaging agent and the NPs selected, and such methodology would be within the remit of a skilled person.

In another embodiment, superparamagnetic iron oxide (SPIO) NPs may be used. SPIO NPs have been used as a feasible means to enhance the contrast of cellular targets in MRI. Some SPIO NPs (for example, Feridex, Endorem and Ferucarbotran) have been approved for human use by the FDA, making them particularly suitable for use in the invention. Commercially available SPIOs, such as Endorem, can be used.

In an embodiment, SPIOs can be encapsulated by an NP as described herein, as a proton magnetic imaging agent. For example, PFCs or SPIOs can be firstly adsorbed by electrostatic or hydrophobic interactions to a polycation and then further complexed with a polyanion to form a NP.

In another embodiment, PFCs or SPIOs can be encapsulated in NPs prepared in supercritical solvents. In this case, PFCs can be used as emulsifying agents and simultaneously be incorporated in the NPs (Reverchon E. (1999) J Supercrit Fluid 15: 1-21).

In another embodiment, PFCs or SPIOs can be adsorbed or encapsulated in carbon nanotubes.

In another embodiment, the NP is itself formed by a fluoropolymer (Du et al. (2008) Biomacromolecules 9(10): 2826-33).

### 3. Inclusion of a Cationic Agent

The NP conjugates of the invention include a cationic agent at, or on, the surfaces of the NPs. This is believed to enhance uptake of the NPs by the target cells. Where the NPs are to be complexed with genetic material as the therapeutic agent, the positively charged NPs are believed to interact electrostatically with the negatively charged DNA/RNA molecules, which not only facilitates complexation of the NP-therapeutic conjugate, but which may also protect the latter from enzymatic degradation and mediate cellular entry. Preferably, the cationic agent may be a polycationic agent.

Suitable polycationic agents for use in the invention include chitosan, peptides (such as poly(L-lysine)), peptide derivatives (such as poly(L-lysine)-palmitic acid), polyethylenimine, poly(amido ethylenimine) (Jeong et al. (2007) Biomaterials 28: 1912-1917), poly(amido amine)s (Lin et al. (2007) Bioconjugate Chem 18: 138-145) and poly(disulphide amine)s (Ou et al. (2009) Biomaterials 30: 5804-5814). A preferred polycationic agent is protamine sulphate (PS).

NPs, prepared as detailed above, may be coated with the chosen cationic agent. Alternatively the cationic agent may be attached to the surface of the NPs, for example by covalent attachment. In embodiments of the invention, a NP formulation may include a mixture of coated and uncoated NPs.

Suitable methods for including cationic agents in the NPs of the invention are as follows.

In one embodiment, the carboxyl groups of a PLGA NP, for example, can be reacted with carbonyldiimidazole, an active carbonylating agent that contains two acylitnidazole leaving groups. Carbonyldiimidazole reacts with carboxylic acids under non-aqueous conditions to form N-acylimidazoles of high reactivity. An active carboxylate can then react with the amine groups of PS, where this is included as a polycationic agent, to form amide bonds.

Alternatively, the carboxyl groups of a PLGA NP can be reacted with carbodiimides. Carbodiimides are used to mediate the formation of amide linkages between a carboxylate and an amine.

In another embodiment, the cationic agent can be adsorbed to the surface of the NP using layer-by-layer (LBL) techniques.

A preferred method for coating NPs with PS is as follows. NPs (1 mg/ml) and PS (1 mg/ml) may be incubated for 10 minutes under agitation, at room temperature. After the incubation period, the NPs may be dialysed (molecular weight cut-off of 50 kDa) against distilled water and freeze-dried.

If SPIO NPs are used, the NPs may be coated with PS substantially as described above, but, after the incubation period, the NPs may be washed by centrifugation with distilled water in cycles of maximum centrifugation speed for 15 minutes, later re-suspended in distilled water and freeze-dried.

These methods may be adapted depending upon the materials used to make the NPs, which adaptation will be within the remit of the skilled person.

### 4. Inclusion of a Therapeutic Agent

The NPs described herein are suitable for use with any therapeutic agent. The therapeutic agent may be encapsulated by the NPs or it may be attached to a surface or surfaces thereof to form a conjugate.

In some cases, the encapsulation of the therapeutic agent is advantageous, as higher concentrations of a drug can be encapsulated than attached at the surface. Drugs can also be delivered at a sustained rate when encapsulated compared to when simply immobilised at an NP's surface.

In other cases, the immobilisation of small concentrations of a therapeutic agent at the surfaces of the NPs can be advantageous. This may be the case when (i) the drug is very active at small concentrations, such as is the case for plasmid DNA, siRNA, miRNA, growth factors etc., (ii) the drug is very sensitive to encapsulation processes, such as those requiring mixing by sonication or homogenisation, or (iii) the drug can be recognised by a cell receptor, such as a growth factor receptor etc.

Suitable methods for encapsulating therapeutic agents inside NPs are as follows.

PLGA may be dissolved in a suitable solvent and an aqueous solution of the selected therapeutic agent plus any excipients (such as serum albumin, magnesium hydroxide etc.) may be added. Such excipients are important to increase the stability of the drug during the encapsulation process (Ferreira et al. (2007) Biomaterials 28: 2706-2717). Typically, the resulting mixture is sonicated or homogenised, at a power dependent upon the ultimate size of NPs desired. This primary emulsion may then be added to an aqueous solution of PVA and homogenised for sufficient time to create a second emulsion. The NPs may then be washed by centrifugation or dialysis, and lyophilised (Nkansah et al. (2008) Biotechnol Bioeng 100(5): 1010-1019).

Another method for encapsulating therapeutic agents inside NPs is via a single emulsion (SE) method. In this case, an aqueous solution of the therapeutic agent and any excipients is mixed with a PLGA solution (in an organic solvent or a mixture of organic solvents). The resulting mixture may then be added dropwise to an aqueous solution of PVA. After a suitable number of hours, the NPs are washed by centrifugation and resuspended in water (alternatively, a dialysis process can be used). The NPs may then be lyophilised.

Another method for encapsulating therapeutic agents within NPs is using the complexation method. Typically, this method is used to encapsulate drugs in NPs that are formed by the electrostatic interaction of polycations with polyanions. The therapeutic agent may be complexed to one of the polymeric components of the NP (i.e. the polycation or the polyanion) and then the complex of drug-polymer may be further complexed with the other NP component (Maia et al. (2011) ACS Nano 5(1): 97-106).

If, however, the selected therapeutic agents are to be attached at the surface of the NPs, the NPs may suitably be immersed in a solution of the therapeutic agent and allowed to complex under appropriate incubation conditions.

For example, LBL techniques can be used to functionalise the surfaces of the NPs and complex the therapeutic agent thereto (Labouta et al. (2010) Int J Pharm 395: 236-242). LBL techniques have been used to tailor the surface of NPs and to design drug delivery systems with controlled release, modulated via layer thickness and composition. Drugs, including dexamethasone, paclitaxel, tamoxifen and siRNA, have been successfully attached to the surface of NPs using LBL techniques (Agarwal et al. (2008) J Control Release 128: 255-260; Elbakry et al. (2009) Nanoletters 9(5): 2059-2064). In addition, LBL NPs with a pH-sensitive outer stealth layer can be used for accumulation in hypoxic tumor regions (Poon et al. (2011) ACS Nano 5(6): 4284-4292).

The therapeutic agent may be a protein, peptide, chemical compound, genetic material (i.e. an oligonucleotide) or any other active molecule.

The intracellular delivery of proteins can be an alternative approach to genetic material to manipulate cellular function. Proteins have key roles in cellular processes and thus are good therapeutic agents. However, due to poor cellular permeability and proteolysis of most proteins, intracellular delivery of proteins is challenging. NPs, as described herein, may play an important role in the protection of proteins from enzymatic degradation and pH hydrolytic effect. Thus, the proteins might be delivered fully functional to integrate and complete the deficient protein machinery inside the cell.

Examples of proteinaceous therapeutic agents that may be delivered intracellularly by the NPs described herein include enzymes (such as glucose oxidase, glucose-6-phosphate dehydrogenase, hexokinase, β-galactosidase, β-glucuronidase, glucocerebrosidase, α-mannosidase, amiloglucosidase, hexoseaminidase A, peroxidase, β-D-fructofuranosidase, neuraminidase, superoxide dismutase, catalase, asparaginase, caspase, DNase, cytochrome oxidase, ATPase and dextranase, among others); peptides (such as thymosin β4, GNRH/LHRH agonists (leuprorelin, goserelin), sandostatin (somatostatin analogue), glatiramer (immunomodulator peptide), salmon calcitonin, desmopressin, bivalirudin and eptifibatide (platelet aggregation inhibitors) and enfuvirtide (HIV cell entry inhibitor), among others); and protein modulators of signaling cascades such as the Wnt pathway, Notch pathyway, TGFβ pathway, Hedgehog pathway and Hippo pathway, among others.

Alternatively, the therapeutic agent may be a small molecule, such as daunorubicin, doxorubicin, vincristine, paclitaxel, amphotericin B, morphine, dexamethasone, retinoic acid and histamine, among others. Increasing the specificity of intracellular delivery of small molecules would not only reduce side effects but also the necessary amount of drug and, consequently, costs. It is possible to predict the interaction of the particles with the cell membrane using computer simulations and such developments are beginning to shed light on the intracellular interaction partners of targeted NPs. For example, SPIO-NPs complexed with a known mitochondrial targeting peptide have been characterised after cellular internalisation by mass spectrometry in order to identify the intracellular proteins that interact with them on their way to the mitochondria (Salaklang J et al (2008) Angew Chem Int Ed Engl 47: 7857-7860). The NPs described herein may similarly increase the specificity of intracellular delivery of small molecules. This could be advantageous, particularly for anticancer drugs and such like, where minimising the potential side effects is key.

In a preferred embodiment, the therapeutic agent may be genetic material (i.e. an oligonucleotide), such as DNA, RNA, short interference RNA (siRNA) or miRNA. Particularly preferred are complexes of NPs and miRNAs. Such molecules are referred to herein as "NP-miRNAs".

miRNAs are short, single-stranded, non-coding RNA molecules found in eukaryotic cells. Being post-transcriptional regulators of gene expression, their binding to complementary sequences on target messenger RNA (mRNA) transcripts usually results in translational repression or target degradation and gene silencing, but possibly results in positive regulation (transcriptional and translational activation) (Bartel DP (2004) Cell 116: 281-297; Bartel DP (2009) Cell 136 (2): 215-233; Wahid F et al. (2010) Biochim Biophys Acta 1803: 1231-1243).

As compared to classical drugs, miRNAs can regulate many target genes and influence a whole gene network. It is believed that the human genome may encode over 1000 miRNAs, which may target about 60% of mammalian genes, and that they are abundant in many human cell types (Bentwich I et al. (2005) Nat. Genet. 37 (7): 766-70; Lewis BP et al. (2005) Cell 120 (1): 15-20; Friedman RC et al. (2009) Genome Res. 19 (1): 92-105; Lim LP et al. (2003) Genes Dev. 17 (8): 991-1008). miRNAs are thus likely to be involved in many, if not most, biological processes.

Aberrant expression of miRNAs has been implicated in numerous disease states, and miRNA-based therapies are under investigation (Trang P et al. (2008) Oncogene 27 Suppl 2: S52-S57; Li C et al. (2009) AAPS j 11 (4): 747-757; Fasanaro P et al (2010) Pharmacol Therapeut 125 (1): 92-104). Recent studies have begun to unveil powerful therapeutic roles for miRNA in numerous forms of ischaemic diseases (Fasanaro P et al. (2010) Pharmacol Therapeut 125 (1): 92-104). As an example, miR-210 has been identified as a key player of endothelial cell response_to low oxygen tension (Fasanaro P et al. (2008) j Biol Chew 283: 15878-15883), regulating mitochondrial free radical response to hypoxia (Favaro E et al. (2010) PLoS One 5: e10345). It is also a cell pro-survival factor, upregulating several angiogenic factors, inhibiting caspase activity and preventing cell apoptosis (Hu S et al. (2010) Circulation 122: S124-131). miR-132 has been reported to induce endothelial cell proliferation and endothelial tube formation in a three-dimensional collagen matrix (Anand A. et al. (2010) Nat Med 16(8): 909-914). miR-424 stabilises hypoxia-inducible factor 1α and plays an important physiological role in post-ischaemic vascular remodeling and angiogenesis (Ghosh G et al. (2010) J Clin Invest 120: 4141-4154). AmiR-92a induces angiogenesis *in vitro* and *in vivo* by regulating the expression of the integrin subunit α 5 (Bonauer A et al. (2009) Science 324: 1710-1713).

However, pharmacokinetics and cell penetration represent potential obstacles to therapeutic miRNA manipulation strategies using miRNA formulations. Several strategies have been reported for the *in vivo* delivery of miRNAs, including (i) chemical modification, (ii) liposomes, and (iii) adeno-associated virus or lentivirus (Shi MA at al. (2010) Sci Signal 3: pe40; Kota J et al. (2009) Cell 137: 1005-1017). Nevertheless, so far, there is no formulation for the *in vivo* delivery of miRNA, with simultaneous control in tracking, pharmacokinetics and internalisation.

The NPs described herein are believed to be particularly efficacious in this regard. The NPs described herein can mediate the intracellular delivery of therapeutically useful miRNAs, notably miR-424, miR-132 and antagomir92a (AmiR-92a). As demonstrated herein, such miRNAs can exert a pro-survival effect in cells exposed to hypoxia, *both in vitro* and *in vivo.*

The NPs described herein are also believed to be beneficial for use with other types of genetic material. Genes can be delivered by viral vectors (including retroviruses, lentiviruses and adenoviruses), non-viral vectors (including polymers and lipids) or physical delivery methods such as electroporation and nucleofaction. However, these are not without their disadvantages. Viral vectors have a risk of toxicity, immunogenicity, insertional mutagenesis and high manufacturing costs, for example. The NPs described herein are believed to be free from such disadvantages.

miRNAs for use with NPs as described herein may be natural or synthetically produced. Suitable methods will be known to those skilled in the art. For example, miR-424 (Ambion), miR-132 (Ambion) and AmiR-92a (Exiqon) may be obtained commercially. All miRNAs, at any stage of development, can be used in the described NPs; thus, pre-miRNA or mature miRNA may be used, for example.

NPs as described here in may be complexed with miRNAs by incubating the NPs with the miRNAs for a suitable time and at a suitable temperature. For example, in one preferred embodiment, NP-PFCEs may be weighed, sterilised under ultraviolet (UV) light for 30 min, and resuspended in EGM-2 serum-free medium to yield a final concentration of 500 µg/ml. The suspension of NPs may then be dispersed by ultrasound (2 x 10 s; BRANSON 2510) and miRNAs may be added (at a concentration of 100 or 200 nM, for example) and allowed to complex to the NPs for 1 h at 37 °C, with intermittent agitation.

Such methods may be adapted depending upon the materials used to make the NPs and the chosen therapeutic agent, which adaptation will be within the remit of the skilled person.

### 5. Inclusion of a Cell Trafficking Agent

Therapeutic agents act at different cellular organelles. For example, DNA is processed at the cell nucleus, siRNA at the cytoplasm or cellular organelles and small inhibitors for the treatment of lysosomal storage disorders at the lysosome. Drug-containing NPs may therefore need to be directed to precise locations within the cell, in order to lead to the desired magnitude and duration of the drug effects.

Most of the current NP formulations are not designed to target specific organelles in the cell. However, NPs can be decorated with organelle-specific targeting moieties to deliver their content at specific organelles; for example, Tat peptide can be used to target the nucleus (de la Fuente JM (2005) Bioconjug Chew 16: 1176-1180), mitochondrial structural protein (MSP) can be used to target the mitochondria (Hoshino A et al. (2004) Microbiol Immunol 48: 985-994) and an antigenic peptide modified with an endoplasmic reticulum (ER)-insertional sequence can be used to target the ER (Matsuo K et al. (2007) Biochem Biophys Res Commun 362: 1069-1072).

The NP conjugates described herein may thus be adorned with a cell trafficking agent, such as a nuclear localisation signal, a mitochondrial localisation signal, an ER signal peptide, an ER retrieval sequence or such like, as is described in the art.

An alternative strategy, however, is to avoid release of the NPs from the endosomal compartment and to use the endomembrane intracellular trafficking system. In other embodiments, therefore, a trafficking agent is not included in the NPs as described herein. In this regard, recent data have indicated that exosomes, which are vesicles released from the endolysosomal pathway, contain RNA and miRNAs; for example, approximately 121 miRNAs have been found in exosomes (Valadi et al. (2007) Nat Cell Biol 9(6): 654-659). Furthermore, recent studies have indicated that these exosome-like vesicles contain single-stranded, mature miRNAs in addition to GW182 and AGO2, which are the two main components of the RNA-induced silencing complex (RISC) (Gibbings et al. (2009) Nat Cell Biol 11(9): 1143-1149). Therefore, the NP formulations described herein offer clear advantages over other formulations (e.g. siPORT transfection agent (Ambion)), as they may be retained in the endolysosomal compartment. Thus, in an embodiment, the NPs described herein are not released into the cell cytoplasm following their delivery into cells.

In summary, therefore an NP conjugate can be prepared by coating or attaching a cationic agent to the surface of a suitable NP, and incorporating a therapeutic agent into the conjugate. The therapeutic agent may be incorporated by any suitable means. It may be complexed to the NP, for example by complexation with the cationic agent, or it may be encapsulated by the Nip. In a preferred embodiment, the NP conjugate may also include an imaging agent. For example, an imagining agent may be encapsulated in the NP, or may be attached to a surface thereof.

### 6. Characterisation of NPs

As already mentioned, the kinetics of cellular attachment and internalisation is affected by NP size, shape, charge and surface chemistry/bioactivity.

NPs for use as described herein have one or more dimensions of from about 1 nm to about 1000 nm, and preferably from about 1 nm to about 500 nm. The NPs may be fine particles, that is to say, they may have one or more dimensions of from about 100 nm to about 500 nm. For example, the NPs may have an average diameter of about 100-250, 200-350, 300-450, 250-500, 400-500 or 200-400 nm. The NPs may be ultrafine particles, that is to say, they may have one or more dimensions of from about 1 nm to about 100 nm. For example, the NPs may have an average diameter of about 1-25, 25-50, 50-75, 75-100, 20-40, 40-60 or 60-80 nm. The NPs may be a mixture of fine and ultrafine particles. For example, the NPs may have an average diameter of about 25-125, 50-150, 75-200, 50-250 or 75-400 nm.

NPs for use as described herein may be of any shape and surface morphology. For example, the NPs may be substantially spherical. The surface may be smooth or fissured. The surface may comprise subnanometric striations of alternating anionic and hydrophobic groups (Verma et al. (2008) Nat Mater 7: 588-595).

The NPs described herein have a positive surface charge, owing to their inclusion of a cationic or polycationic agent, such as protamine sulfate. Positive surface charge is believed to be beneficial, as it may promote interaction of the NPs at target cell membranes. In addition, the positive surface charge advantageously facilitates complexation to negatively charged moieties, such as miRNA.

The NPs may be characterised in terms of particle size, shape, morphology, surface charge and such like using standard methods in the art.

For example, particle size may be determined using light scattering, using a Zeta PALS Zeta Potential Analyzer and ZetaPlus Particle Sizing Software, v. 2.27 (Brookhaven Instruments Corporation), for example. In one such embodiment, NPs may be suspended in 1 mM potassium chloride buffer at pH 5.5 (500 µg/ml) and sonicated for short periods (<1 min). Typically, all sizing measurements are performed at 25 °C, and all data are recorded at 90°, with an equilibration time of 5 min and individual run times of 120 s (five runs per measurement).

Particle shape, morphology and size can be evaluated using TEM (JEOL JEM-100 SX microscope at 80 kV) following negative staining with osmium tetroxide, for example. In one such embodiment, an NP suspension (5 µl, 5 mg/ml in water) and osmium tetroxide solution (5 µl, 5% w/v) may suitably be placed on a 400 mesh copper grid with a carbon-coated Formvar membrane. The sample can be dried overnight before examination by TEM.

Other methods for characterising NPs as described herein will be known to the skilled person.

### 7. Cell Uptake of NPs

The problem with many known drug delivery systems is that they are recognised by the human body as foreign and, hence, they become easily opsonised and removed from the circulation long before they have fulfilled their function. The NPs described herein are thus advantageous, as they are able to achieve a better effect of targeted drug delivery systems due to their rapid internalisation by target cells.

It is believed that the NPs described herein may be internalised by the target cells by receptor-mediated endocytosis or some form of active transport. Alternatively, they may enter the cell via a non-specific internalisation process. The cationic agent at, or on the surface of, the NPs is believed to assist in the interaction of the NPs with their target cells, for example, at the appropriate receptor, membrane channel, gate or such like. If a negatively-charged therapeutic agent, such as miRNA, is present at the surfaces of the NPs, this may further assist in the cell surface interaction. For example, the miRNA molecules may themselves target the NPs to their corresponding cell surface receptors.

Once the target cells have internalised the NPs, the latter are believed to be taken up in endosomes. Any early endosomes formed may develop and fuse to form late endosomes.

In the absence of inclusion of an intracellular trafficking agent, the NPs are believed to largely remain at the endosomal compartment over time. Where miRNAs are used as the therapeutic agent, the NP-miRNA complexes may thus remain inside the endosomes. Staying inside endosomes may be advantageous, as it may contribute to more close encounters with potential partner molecules. In this regard, the endomembrane system seems to have a specific way of assessing vesicles and sorting cargoes according to cellular needs. The NPs described herein may thus ride the endomembrane system and be transported to their final destination in a more direct and effective way.

Alternatively, the miRNA molecules may be released from the NP-miRNAs into the cell cytoplasm. This is particularly the case where a trafficking agent is included in the NPs as described herein.

Any cell type may be targeted by the NPs described herein. In a preferred embodiment, the target cells are MNCs or endothelial cells (particularly HUVECs).

Cell uptake of NPs may be achieved *in vitro* as follows.

MNCs, for example, may be obtained from umbilical cord blood (UCB) samples using Ficoll density gradient separation. HUVECs, for example, may be isolated from human umbilical vein using standard procedures in the art.

MNCs, HUVECs or other target cells may be labelled with NPs as herein described using any suitable method. For example, 2 x 10⁶ cells/condition may be labelled with NPs in an amount of between 500 µg/ml and 8 mg/ml for 4 h in serum-free EGM-2 (HUVECs) or serum-free M199 (MNCs). This may be performed in any suitable cell culture vessel.

After incubation, the cells (for example, HUVECs) may be washed, suitably three times, with phosphate buffered saline (PBS) and trypsinised (with 0.2% (v/v) trypsin, for example), to release them from the walls of the culture vessel. MNCs may be washed, suitably three times, with PBS. The cells may be passed through a suitable separating column, such as a MACS column (Miltenyi Biotec), to further separate them from any non-internalised NPs. Cell viability may be determined using trypan blue exclusion, as is standard in the art. The cells, once labelled with NPs in this way, may be resuspended in PBS, frozen at -80 °C and/or freeze-dried, as desired.

If cells are to be transfected (i.e. an NP as described herein is to deliver genetic material, such as miRNA, into the target cells), the target cells (such as HUVECs) may be washed twice with pre-warmed PBS and the suspension of NP-miRNA (or other genetic material) may be added to the cells for incubation at 37 °C for 4 h. Cells are suitably transfected keeping a ratio of 1.25 mg of NP-miRNA (or other genetic material) per million HUVECs.

In a SiPORT transfection procedure, a suspension of the SiPORT transfection agent and the genetic material (for example, miRNAs) may be dispensed onto PBS-pre-washed cells and incubated for 4 h at 37 °C.

Alternatively, the cells can be seeded onto circular 20 mm glass coverslips coated with 0.2% gelatin inside a 24 well plate and left to adhere. The cells may then be incubated with NPs (500 µg/ml) for 4 h. Once the incubations are terminated using a standard procedure in the art, the coverslips may be washed gently, optionally staining for lysosomes (using LysoTracker red DND-99 at 50 nM, 20 min incubation) or mitochondrial activity (using MitoTracker Red CM-Ros, 50 nM, 15 min incubation) may be carried out and the cells may be rewashed as necessary.

In another embodiment, MNCs (1×10⁶ cells) or HUVECs (0.2×10⁶ cells), for example, may be incubated for 4 h in serum free M199 or serum free EGM-2, respectively, containing variable concentration of NPs in a 24 well plate. After 4 h, the HUVECs may be washed with PBS on the plate three times. The cells may then be re-suspended in serum free EGM-2, prior to trypsinisation with 0.2% (w/v) trypsin solution and centrifugation at 1000 rpm for 5 min.

Cell uptake of NPs may be achieved in cells for transplantation in human patients. In human melanoma patients, dendritic cells (15 x 10⁶ cells in 200 ml) labelled with NP-PFCE using methods described herein can be transplanted directly into a lymph node region. Suitable transplantation methods are described elsewhere (de Vries et al. (2005) Nat Biotechnol 23(11): 1407- 1413).

The foregoing methods may be adapted depending upon the particular NPs, therapeutic agents and target cells selected, which adaptation will be within the remit of the skilled person.

Cell uptake of NPs may be achieved *in vivo* as follows.

NPs containing the chosen therapeutic agent can be injected into the brain using stereotaxic surgery. After anaesthesia, an incision into the dorsal aspect of the head can be made, exposing the cranium and the bregma, and a fine dental air-drill can be used to drill a hole in the skull. Slow microinjection equipment can be used to deliver NPs (2-10 µl in mice; 500 µg/ml of NPs containing the therapeutic agent) into the substantia nigra or the brain left ventricle (Bharali et al. (2005) PNAS 102: 11539-11544).
In another embodiment, NPs containing the therapeutic agent can be injected in the bloodstream to target tumours. In this case, NPs can cross endothelial cells and accumulate within tumour cells, a process referred to as enhanced permeation and retention (EPR) (Farokhzad et al. (2006) PNAS 103: 6315-6320).

In another embodiment, NPs containing the therapeutic agent can be injected in the bloodstream to target ischaemic areas. If active targeting is desired, a molecular target must be identified at the desired site of drug action that is substantially enriched relative to the rest of the body, and a ligand to that target must be provided. For example, NPs coated with angiotensin II type 1 (100 µl in mice) may be injected into the right jugular vein, in order to target the infarcted heart (Dvir et al. (2011) Nano Lett 11: 4411-4414).

### 8. In Vitro Cell Tracking

*In vitro* cell tracking is useful for measuring, understanding and manipulating cells, evaluating cellular therapeutics and drug delivery and understanding disease progression.

NP-labelled cells may be tracked *in vitro* using any suitable technique. In a preferred embodiment, the NPs may comprise a fluorine compound that can be tracked by ¹⁹F-MRI, for example.

The inclusion of a fluorine compound, such as PFCE, may be performed as described herein. The target cells may be labelled with the fluorine-containing NPs (for example, NP-PFCEs) and lyophilised, also as described herein. The lyophilised cells may then be characterised by ¹⁹F NMR according to standard procedures in the art. For example, ¹⁹FNMR analyses may be performed in a 600 MHz NMR Varian Instrument. Suitably, NPs (5 mg) are dissolved in a suitable volume of ¹H-dichloromethane (such as 300 µl) and transferred to a 5 mm NMR tube. A sealed 3 mm tube containing trifluoroacetic acid with ¹H-dichloromethane may be inserted in the 5 mm tube and used as an internal reference. A total of 50 scans may be run, with pulses at 90° after a relaxation time of 15 seconds.

Alternatively, confocal microscopy may be used. In this embodiment, target cells may be seeded onto glass coverslips, incubated with 500 µg/ml fluorine-containing NPs (for example, NP-PFCEs), washed gently and, optionally, life stained for lysosomes or mitochondrial activity, as described herein, and rewashed prior to fixation. The cells may then be fixed with 4% (w/v, in water) paraformaldehyde (EMS, Hatfield, USA) for 10 min at room temperature and washed with PBS.

For certain conditions, the slides may be mounted straight away with mounting medium-4',6-diamidino-2-phenylindole (DAPI; Sigma-Aldrich) on a glass slide.

However, in some conditions, cell membrane staining may be performed. In these embodiments, mouse antibodies against human CD31 (Dako, Glostrup, Denmark) or CD45 (BD Biosciences, Spain) may be used to stain the membrane of target cells such as HUVECs or cord blood MNCs. Cells may be washed three times after fixation (MNCs may be washed by centrifugation), the cells may then be blocked with 2% bovine serum albumin (BSA) (Sigma-Aldrich) in PBS for 30 min at room temperature. The CD31/CD45 (both at 1:50 dilution) primary antibody may be added in PBS for 1 h at room temperature followed by 3 washes in PBS. The binding of primary antibodies may be detected with anti-mouse DyLight 649 conjugate (at a dilution of 1:200) Jackson ImmunoResearch, USA). The nucleus of cells may be stained with mounting medium-DAPI. After the indirect labelling, the cells may be examined with a Zeiss Laser Scanning Microscope Meta 510 or such like.

In another preferred embodiment, fluorescence activated cell sorting (FACS) analysis may be used. Target cells, such as MNCs or HUVECs, may be labelled with NPs in a 24-well plate and later washed with PBS, as described herein. The cells may be re-suspended in serum free EGM-2 with mitotracker red CMX-ROS at 50 nM for 15 min at 37 °C in a carbon dioxide incubator. The cells may then be trypsinised with 0.2% (w/v) trypsin solution, centrifuged at 1000 rpm for 5 min, and fixed with 4% paraformaldehyde for 10 min at room temperature. After fixation, the cells may be re-washed and then re-suspended in 500 µl of PBS, ready for FACS analysis. A total of 80,000 events may be recorded per measurement.

MNCs may be transferred to a 2 ml eppendorf and centrifuged for 15 min at 20 °C, 1300 rcfs, then the MNCs may be further washed by using anti-CD45 human microbeads via a Mini MACS system and protocol (Miltenyi Biotec, UK) so as to ensure that only MNCs, and no free NPs, are present in the final solution. The cells may then be incubated with mitotracker (50 nM) for 15 min at 37 °C, washed once with PBS and fixed with 4% paraformaldehyde for 10 min at room temperature. After fixation, the cells may be washed and resuspended in 500 µl of PBS before FACS analysis. A total of 80,000 events may be recorded per measurement.

If the target cells have been transfected, for example, using a SiPORT transfection procedure as described herein, transfection visualisation may be performed under a fluorescence microscope using NP-PFCEs labelled with fluorescein and miRIDIAN microRNA mimic transfection control with Dy547 (Dharmacon).

In another embodiment, fluoresence imaging may be used. For example, sample tubes may be placed inside a FluorVivo 300 (INDEC Biosystems, Santa Clara, USA) or such like. Exposure times are typically between 0.05 and 0.15 s.

Any of the foregoing methods may be adapted depending upon the particular NPs, imaging agents and target cells selected, which adaptation will be within the remit of the skilled person.

### 9. In Vivo Cell Tracking

NPs may also be tracked *in vivo,* by any suitable means, preferably non-invasively. This could be useful in measuring, understanding and manipulating cells *in vivo.* Monitoring *in vivo* cell trafficking by non-invasive means is also critical to improving cellular therapeutics, drug delivery and understanding disease progression. It may be used to optimise the route of delivery, cell localisation, dosage and other factors.

MRI may suitably be used for this purpose, owing to its non-invasive nature, intrinsic anatomic contrast, high resolution and because it does not use ionising radiation. ¹⁹F-MRI, in particular, allows extremely specific detection and quantification of cell numbers directly from *in vivo* image data and, thus, is a preferred procedure. Such *in vivo* imaging enables the localisation, quantity and viability of cellular therapeutics to be carefully monitored.

*In vivo* tracking may be performed in animal models of diseases such as myocardial infarction and hind limb injury, for example. By way of illustration, NPs as described herein can be used to track cardiosphere derived cells (CDCs; also known as cardiac stem cells) and bone marrow-derived cells into rat models of myocardial infarction, as follows.

Rat CDCs may be isolated from hearts of Sprague Dawley rats (Harlan, UK) and cultured as described in Carr et al. (2011) PlosOne 6: 10. CDCs (1x10⁷ cells) at passage 2 may then be labelled with 500 µg/ml of NPs per 400,000 cells for 4 h at 37 °C and 5% CO₂ in explant medium (EM) comprising Iscove's modified Dulbecco's medium (IMDM; Invitrogen) supplemented with 2% foetal bovine serum (FBS; Invitrogen), 1 U/ml penicillin, 1 µg/ml streptomycin and 0.2 mM L-glutamine (Gibco). After the incubation period, cells may be washed three times with PBS and trypsinised. The cells may be re-suspended into 50 µl PBS (Sigma) and placed into U-100 insulin syringes (Becton, Dickinson) maintained in ice until required for injection.

Rat bone marrow derived cells may be isolated from the femur and tibia of sacrificed Sprague Dawley rats (Harlan, UK). The ends of the bones may be cut off and the cavity flushed with PBS to remove whole bone marrow. Total mononuclear bone marrow (MBM) derived cells may be isolated via density gradient centrifugation using Ficoll-Paque Premium (GE Healthcare, UK). MBMs (1.2x10⁷ cells) straight after isolation may be labelled with 500 µg/ml NPs per 1,000,000 cells for 4 h at 37 °C and 5% CO₂ in medium199 (M199) supplemented with 2% FBS (Invitrogen), 1 U/ml penicillin and 1 µg/ml streptomycin (Gibco). After the incubation period, cells may be washed three times with PBS. The cells may be re-suspended into 50 µl PBS (Sigma) and placed into U-100 insulin syringes (Becton, Dickinson & Company) maintained in ice until required for injection.

The left anterior descending (LAD) coronary artery of Sprague Dawley rats (Harlan, UK) may then be occluded using a method described elsewhere (Michael et al. (1995) Am J Physiol 269: H2147-2154). In brief, following anaesthesia, using 2% isoflurane in oxygen, and thoracotamy, the pericardium may be removed and a 5-0 prolene suture placed under the LAD, about 2 mm from the origin. The suture may be tied around a small piece of polyethylene tubing, occluding the LAD, and the chest closed. After 50 minutes, the chest may be re-opened and the tubing removed to allow reperfusion. Ten minutes after ischaemia/reperfusion, cells labelled with NPs with or without miRNAs may be injected over four sites in the peri-infarct region. In sham animals, thoracotamy may be performed but no stitch is placed in the heart. Animals should be allowed free access to standard rodent chow and water throughout the study.

Cell tracking may be measured via ¹⁹F MRI and cardiac function may be measured using ¹H cardiac gated CINE MRI on a horizontal bore 7 Tesla, 300MhZ Varian instrument using VnmrJ software.

As a further illustration, NPs as described herein can be used to track endothelial cells and smooth muscle cells into mice and rabbit hind limb models, as follows. A model of acute ischaemia may be induced in the right hind limb of rabbits and mice by surgical procedures, as previously described in Prompers et al. (2006) NMR Biomed 19:927-953. Briefly, the superficial part of the femoral artery may be ligated under ketamine (Ketalar, Pfizer 0.3 ml/kg) and medetomidine (Domitor, Orion, 0.3 ml/kg) anaesthesia. The re-entry branches for the collaterals growing from the lateral circumflex and deep femoral arteries may be ligated. In this model, the calf region is ischaemic while the thigh remains normoperfused. In a sham operation, all other procedures are similar to the actual operation except that ligatures may be placed loosely around vessels, not blocking blood flow. The rabbits and mice may receive cells labelled with NPs with or without miRNAs, or cells without NPs at all, via intramuscular injection. Intramuscular injection may be performed in the semimembranosus, abductor cruris cranialis, quadriceps femoris, tibialis anterior and gastrocnemius muscles using a 1 ml syringe and a 25-gauge needle under ketamine- medetomidine anaesthesia.

Cell tracking may be measured via ¹⁹F MRI, flow measured using VEVO ultrasound and cellular proliferation using IVIS systems.

Such methods may be adapted depending upon the particular NPs, imaging agents and target cells selected, and the subject to be imaged, which adaptation will be within the remit of the skilled person.

Alternatively, *in vivo* fluorescence imaging may be used. For example, animal models may be placed inside a FluorVivo 300 (INDEC Biosystems, Santa Clara, USA) or such like. Exposure times are typically between 0.05 and 0.15 s. Other techniques for use with NPs as described herein include positron emission tomography (PET), ultrasound and computed tomography (CT), for example.

### 10. Clinical Uses

Many clinical applications of the NPs described herein are envisaged. For example, MNCs, HUVECs or bone marrow cells labelled with NP-miRNAs as described herein could potentially be administered to patients suffering a myocardial infarction or other ischaemic disease. It is thought that cancer patients may also benefit from treatment with NP-miRNAs as described herein, as a subset of the latter are believed to be anti-angiogenic.

In order to facilitate understanding of the appended examples, the following acronyms will be used:

| **Acronym** | |
|---|---|
| NPs | Nanoparticles |
| PLGA | Poly(lactic acid-co-glycolic acid) |
| PLGA-PFCE | Poly(lactic acid-co-glycolic acid)-Perfluoro-1,5-crown ether nanoparticle conjugate |
| *PLGA-PFCE | Fluorescent-labelled Poly(lactic acid-co-glycolic acid)-Perfluoro-1,5-crown ether nanoparticle conjugate |
| PS | Protamine sulphate |
| PLGA-PFCE-PS | Conjugate of Poly(lactic acid-co-glycolic acid)-Perfluoro-1,5-crown ether nanoparticles coated with protamine sulphate |
| *PLGA-PFCE-PS | Conjugate of fluorescent-labelled Poly(lactic acid-co-glycolic acid)-Perfluoro-1,5-crown ether nanoparticles coated with protamine sulphate |
| SPIO | Superparamagnetic iron oxide |
| *SPIO | Fluorescent-labelled superparamagnetic iron oxide nanoparticles |
| SPIO-PS | Superparamagnetic iron oxide nanoparticles coated with protamine sulphate |
| *SPIO-PS | Fluorescent-labelled superparamagnetic iron oxide nanoparticles coated with protamine sulphate |
| PLGA-PFCE-PS-miRNA | Conjugate of Poly(lactic acid-co-glycolic acid)-Perfluoro-1,5-crown ether nanoparticles coated with protamine sulphate and complexed with miRNA |
| *PLGA-PFCE-PS-miRNA | Conjugate of fluorescent-labelled poly(lactic acid-co-glycolic acid)-Perfluoro-1,5-crown ether nanoparticles coated with protamine sulphate and complexed with miRNA |
| FITC | Fluorescein isothiocyanate |
| FITC-PLGA-PFCE | Conjugate of Poly(lactic acid-co-glycolic acid)-perfluoro-1,5-crown ether nanoparticles labelled with fluorescein isothiocyanate |

### Examples

The invention will now be described by way of illustration only in the following examples:

### Example 1: Isolation of MNCs from UCB

All human UCB samples were collected from donors, who signed an informed consent form, in compliance with Portuguese legislation. The collection was approved by the ethical committee of Hospital Infante D. Pedro. The samples were stored in sterile bags containing 35 ml of citrate-phosphate-dextrose anticoagulant solution. MNCs were obtained from UCB samples after Ficoll (Histopaque-1077 Hybri Max; Sigma-Aldrich, St. Louis, USA) density gradient separation. MNCs were immediately used for experiments without further treatment.

### Example 2: Preparation of fluorescent-labelled NPs

### 2.1 Preparation of fluorescent-labelled NPs containing fluorine-imaging agent [*PLGA-PFCE]

PLGA NPs were prepared by a single emulsion protocol. PLGA (Resomers® 502 H; 50:50 lactic acid: glycolic acid) (Boehringer Ingelheim) was covalently conjugated to fluoresceinamine (Sigma-Aldrich) according to a protocol reported by Horisawa et al. (2002) Pharm Res 19: 132-139. The fluoresceinimine acts as a simple reporter to follow NP delivery into cells. The NPs were prepared by dissolving the fluorescent-labelled PLGA (100 mg) in a solution of dichloromethane: trifluoroethanol (1:8, 6.3 ml) containing PFCE (100 mg) (Fluorochem, UK). This solution was then added dropwise to a PVA solution (40 ml, 5% w/v in water) and stirred for 3 hours. The *NPs were then centrifuged and washed with distilled water before freeze-drying.

### 2.2 Preparation of fluorescent-labelled SPIO NPs

Fluorescent-labelled SPIO NPs were used as a control. SPIO NPs commercialized as Endorem [Chu W. et al. (1995) Magnetic Resonance Imaging 13: 661-674] (size 80-160 nms) were kindly donated by Guebert. The SPIO NPs were labelled with fluoresceinimine primarily as reported in Lee et al. (2008) Adv Mater Deerfield 20: 2512-2516, with some modifications. Briefly, SPIO NPs (approximately 10 mg in 1 ml of 10 mM citrate buffer pH 5.5) were incubated with sodium periodate (10 mg) for 2 hours at room temperature. The solution was ultra-centrifuged at 41,000 rpm for 15 minutes. The supernatant was discarded and the pellet was washed twice with distilled water. The remaining pellet was added to a solution of fluoresceinamine (2 mg/ml) in cyanoborohydride pH 8 (50 mM) and reacted for 2 hours at room temperature. The NP suspension was then ultra-centrifuged at 41,000 rpm for 15 minutes and once again washed with distilled water, re-suspended and lyophilized.

### Example 3: Coating of NPs with protamine sulphate (PS)

In some cases, NPs were coated with PS as described below.

### 3.1 Coating of fluorescent-labelled PLGA-PFCE NPs

*PLGA-PFCE NPs prepared in example 2.1 above (1 mg/ml) were incubated with PS (1 mg/ml) for 10 minutes under agitation, at room temperature. After the incubation period, the NPs were dialysed (MWCO of 50 kDa) against distilled water and freeze-dried.

### 3.2 Coating of fluorescent-labelled SPIO NPs

*SPIO NPs prepared in example 2.2 (1 mg/ml) above were incubated with PS (1 mg/ml) for 10 minutes under agitation, at room temperature. After the incubation period, the NPs were washed by centrifugation with distilled water in cycles of maximum centrifugation speed for 15 minutes, later re-suspended in distilled water, and freeze-dried.

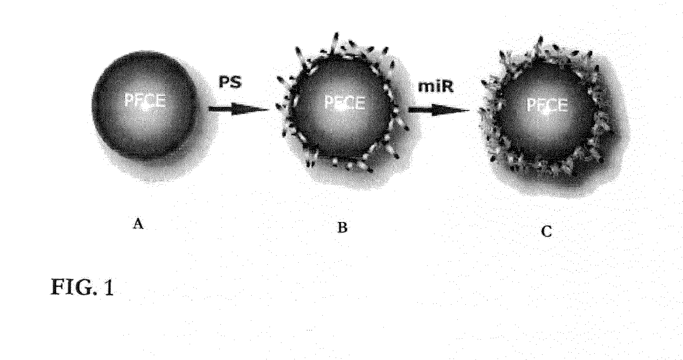

A schematic representation of the NP preparation and coating described in Examples 2 and 3 above is illustrated in Figure 1. In Step (A) of Figure 1, the NPs are formed by PLGA encapsulating PFCE. In Step (B) of Figure 1, the NPs are then coated with a polycationic agent, PS, which has a dual role: (i) to facilitate cell internalization and (ii) to mediate the complexation of miRNAs. The complexation of the coated NPs to miRNAs (Step C) is discussed in Example 8 below.

### Example 4: Characterisation of NPs

### 4.1 Dynamic Light Scattering

Particle size was determined using light scattering via Zeta PALS Zeta Potential Analyzer and ZetaPlus Particle Sizing Software, v. 2.27 (Brookhaven Instruments Corporation). NPs suspended in 10 mM potassium chloride (KCl) buffer pH 5.5 (500 µg/ml) and sonicated for short times (<1 minute) were used. Typically, all sizing measurements were performed at 25 °C, and all data were recorded at 90°, with an equilibration time of 5 minutes and individual run times of 120 s (5 runs per measurement). The average diameters described in this work are number-weighted average diameters. The zeta potential of NPs was determined in a 10 mM KCl pH 5.5 solution, at 25 °C. All data were recorded with at least 6 runs with a relative residual value (measure of data fit quality) of 0.03.

The results of this analysis are shown in Table 1 below:

**Table 1:**

| **NP** | **Diameter (nm)** | **Polydispersion Index (PDI)** | **Zeta potential (mV)** | **Recovery yield**(%)** |
|---|---|---|---|---|
| *PLGA | 169.8 ± 7.1 | 0.680 | -9.3±2.8 | 80.5±8.7 |
| *PLGA-PFCE | 212.9 ± 14.3 | 0.244 | -9.7±0.7 | 75.3±8.6 |
| *PLGA-PFCE-PS | 218.0±9.3 | 0.381 | +7.0±1.7 | 83.9±3.1 |
| SPIO | 89.2±3.4 | 0.267 | -2.9±0.9 | n/a |
| *SPIO | 82.7±2.5 | 0.271 | +3.8±0.1 | n/a |

| | | | | |
|---|---|---|---|---|
| All NPs marked * were fluorescently labeled with fluoresceinimine as described in examples 2.1 and 2.2. ** The recovery yield of NPs after production was calculated according to the following equation: recovery yield = (NP weight x 100)/(initial PLGA weight + mass of PFCE used). | | | | |

This dynamic light scattering analysis showed that PLGA NPs without PFCE had an average diameter of 170 nm and a negative (∼ -9 mV) zeta potential. The encapsulation of PFCE in the NPs increased their average diameter from 170 to 213 nm.

### 4.2 Transmission electron microscopy (TEM) analysis

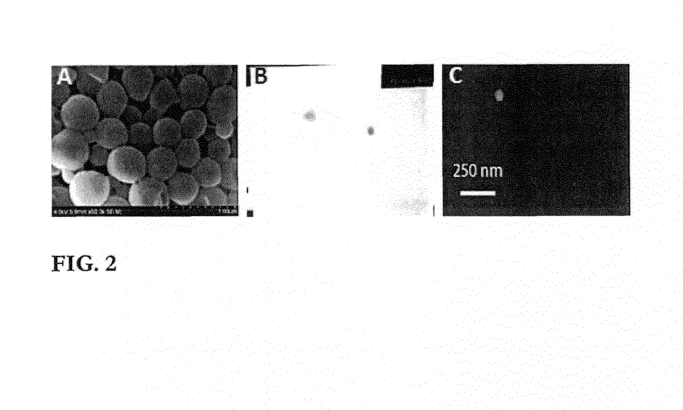

The morphology and size of the NPs was also evaluated by TEM (JEOL JEM-100 SX microscope at 80 kV) following negative staining with osmium tetroxide. A NP suspension (5 µl of PLGA-PFCE, 5 mg/ml in water) and osmium tetroxide solution (5 µl, 5% w/v) were placed on a 400 mesh copper grid with a carbon-coated Formvar membrane. The sample was then dried overnight before examination by TEM. The results of the TEM analysis of the PLGA-PFCE NPs are shown in Figure 2.

### 4.3 Mass loss of PLGA-PFCE

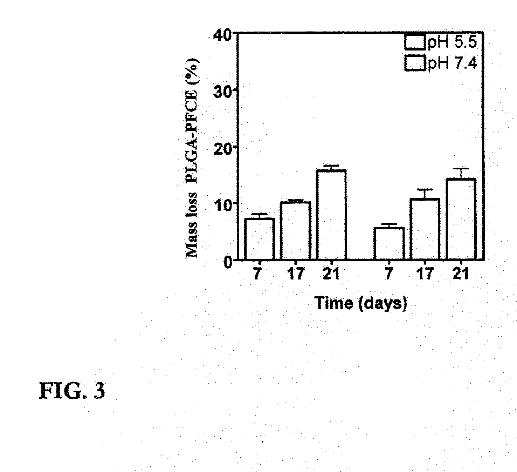

Mass loss of PLGA-PFCE NPs suspended in buffered solutions at pH 5.5 and 7.4 for several days, at 37 °C, was determined as followed. NPs (20 mg/ml) were re-suspended in PBS (pH 7.4) or KCl (pH 5.5) buffers and dialyzed against the corresponding buffers at 37 °C. At specific time points (days 7, 17 and 21) the NP suspension was lyophilized and percentage of mass loss was calculated by subtracting the final mass from the initial mass. Results are shown as mean ± SD, n=3 in Figure 3.

### 4.4 Diameter of NPs over time

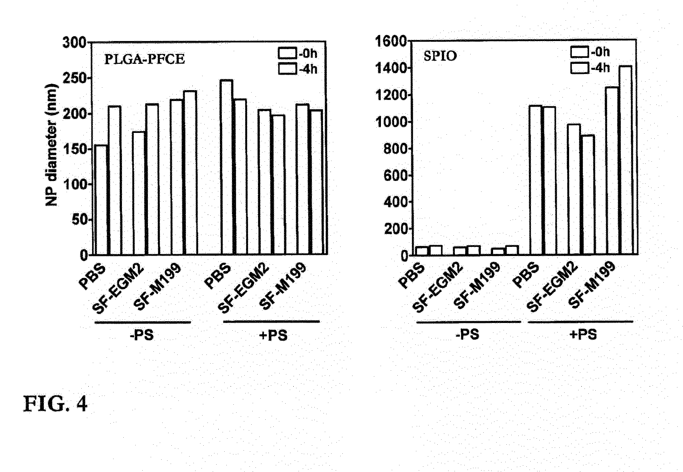

The diameter of PLGA-PFCE and SPIO NPs with and without the PS coating was measured. The NPs were suspended in PBS or culture media (serum free M199 and serum free EGM2 media) and the diameter was measured at time 0 and after 4 hours. The NPs were suspended at 1 mg/ml in the corresponding media and sonicated for 15 seconds before measurement (t = 0). Alternatively, the NPs were suspended at 1 mg/ml in the corresponding media and sonicated for 15 seconds, then incubated for 4 hours at 37 °C before measurement (t = 4 hours). The counts per second (Kcps) during the measurements were between 300-400 Kcps. The results are expressed as average ± SD (n=3) in Figure 4.

### 4.5 Quantification of fluorine in NPs by ¹⁹F-NMR

¹⁹F NMR analyses were performed in a 600 MHz NMR Varian Instrument. PLGA-PFCE NPs (3 mg) were dissolved in dichloromethane (300 µl) and transferred to a 5 mm NMR tube. A sealed 3 mm tube containing trifluoroacetic acid (TFA) with ¹H-dichloromethane was inserted in the 5 mm and used as an internal reference. A total of 50 scans were run, with pulses at 90° after a relaxation time of 15 seconds.

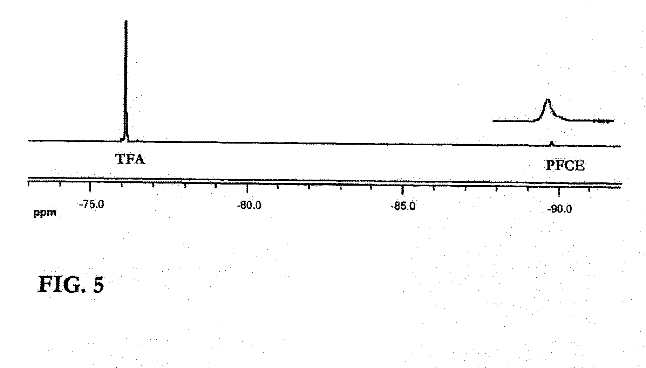

The ¹⁹F NMR spectrum of the PLGA-PFCE NPs dissolved in dichloromethane is shown in Figure 5. The concentration of fluorine was calculated from the ratio of the peak area at δ -89 ppm (fluorine in PFCE) and the one at -76 ppm corresponding to a known concentration of the internal reference (TFA). The results are shown in Table 2 below.

**Table 2:**

| **NP Formulation** | **PFCE (µg per mg PLGA)** | **Encapsulation efficiency (%, w/w)*** | **Fluorine concentration (µg of F per mg of NPs)** |
|---|---|---|---|
| PLGA-PFCE | 176.5 | 4.9% | 98.0 |
| (ratio of 3.6 mg PFCE: 1 mg PLGA) | | | |
| PLGA-PFCE | 111.0 | 13.8% | 65.3 |
| (ratio of 0.8 mg PFCE: 1 mg PLGA) | | | |

| | | | |
|---|---|---|---|
| *Encapsulation efficiency (% w/w) = (experimental amount of PFCE in PLGA-PFCE NPs × 100) / initial amount of PFCE. | | | |

It can be seen from Table 2 that the encapsulation efficiency assessed by ¹⁹F NMR analysis was between 4.9% and 13.8%. The PLGA-PFCE formulation has between 111.0 and 176.5 µg per mg of PLGA depending on the initial PFCE concentration used for the preparation of the PLGA-PFCE NPs.

### 4.6 Quantification of fluorine in NP-labelled cells by ¹⁹F-NMR

Cells (2 × 10⁶ cells/condition) were labelled with the PLGA-PFCE NPs (between 500 µg/ml and 8 mg/ml) for 4 hours in serum free EGM-2 (HUVECs) or serum free M199 (MNCs). After incubation, HUVECs were washed 3 times with PBS and trypsinised (0.2% trypsin), while MNCs were washed 3 times with PBS and then passed through a MACS column (Miltenyi Biotec) to further separate the cells from the non-internalized NPs. At the end, all cells were counted with trypan blue, resuspended in PBS, frozen at 80 °C and freeze-dried. The lyophilised cells were then dissolved in ¹H-dichloromethane (300 µl), transferred to a 5 mm NMR tube and characterized by ¹⁹F NMR as described in Section 4.6 above.

### Example 5: Characterisation of intracellular delivery

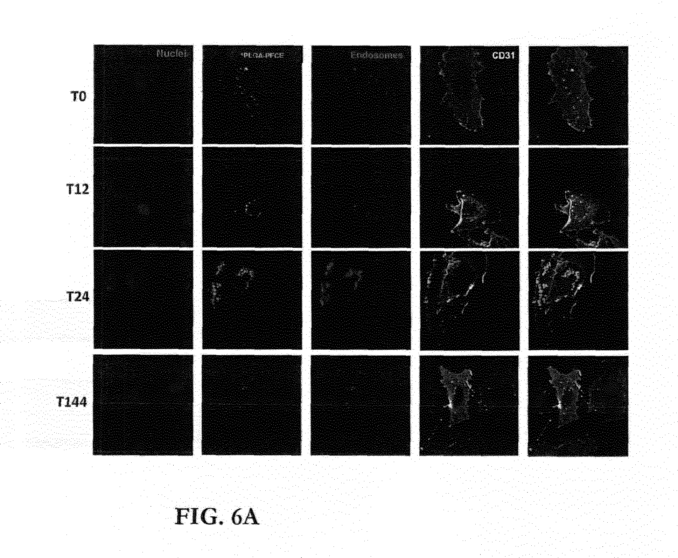

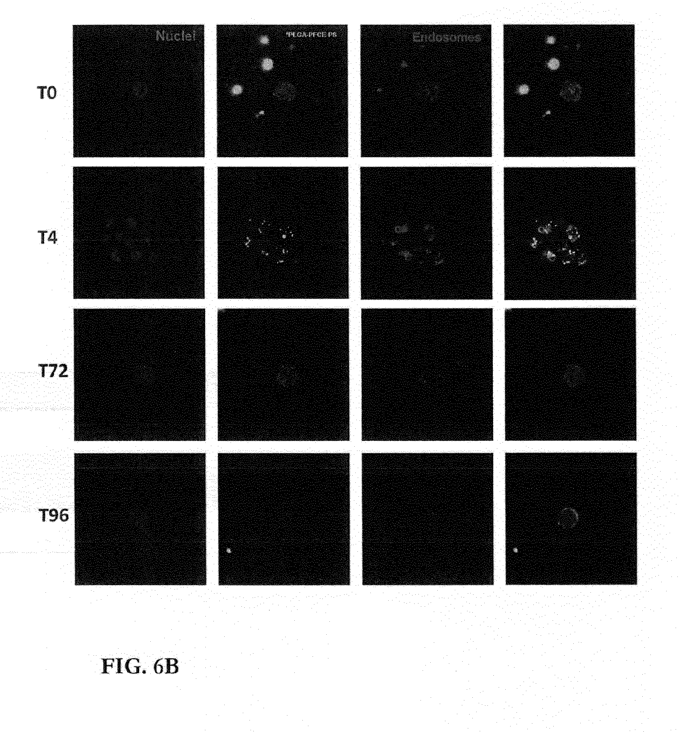

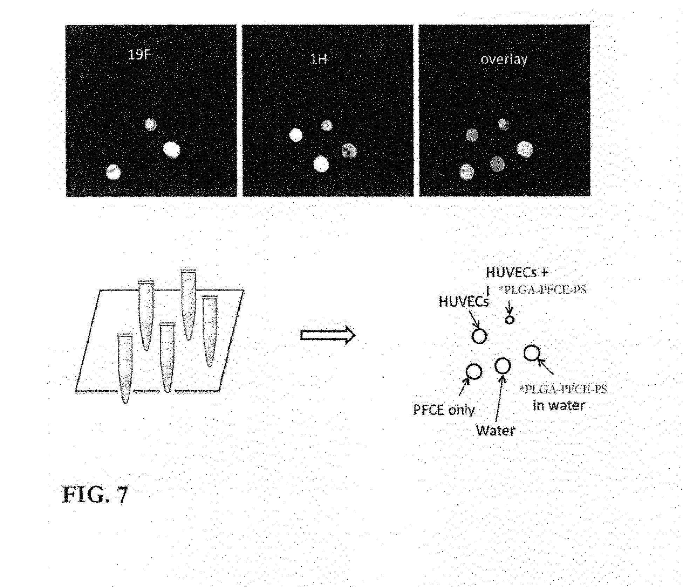

To characterise intracellular delivery of the fluorescent-labelled PLGA-PFCE NPs into the cytoplasm of human umbilical vascular endothelial cells (HUVECs) or cord blood MNCs, confocal laser scanning microscopy and MRI analyses were performed. * SPIO NPs were used as a control. Results are shown in Figures 6 and 7.

### 5.1 Confocal imaging

Cells were seeded onto circular 20 mm glass coverslips coated with 0.2% gelatin inside a 24 well plate and left to adhere. The cells were then incubated with 500 µg/ml of *PLGA-PFCE NPs or *SPIO NPs for 4 hours in serum free EGM-2 (SF-EGM-2; HUVEC loading medium) or serum free M199 (SF-M199, MNC loading medium).

Once the incubations were terminated the coverslips were washed gently, in some conditions live staining for endolysosomal vesicles (using LysoTracker red DND-99 at 50 nM, 20 minute incubation) or mitochondrial activity (using MitoTracker Red CM-Ros, 50 nM, 15 minute incubation) was done and rewashed prior fixation. Then, cells were fixed with 4% (w/v, in water) paraformaldehyde (EMS, Hatfield, USA) for 10 minutes at room temperature and then washed with PBS. For certain conditions, the slides were mounted straight away with mounting medium-4',6-diamidino-2-phenylindole (DAPI; Sigma-Aldrich) on a glass slide. However, in some conditions, cell membrane staining was performed. In this case, mouse antibodies against human CD31 (Dako, Glostrup, Denmark) or CD45 (BD Biosciences, Spain) were used to stain the membrane of HUVECs or MNCs. Cells were washed 3 times after fixation (MNCs were washed by centrifugation), the cells were then blocked with 2% bovine serum albumin (BSA) (Sigma-Aldrich) in PBS for 30 minutes at room temperature. The CD31/CD45 (both at 1:50 dilution) primary antibody was added in PBS for 1 hour at room temperature followed by 3 washes in PBS. The binding of primary antibodies was detected with anti-mouse DyLight 649 conjugate (at a dilution of 1:200) (Jackson ImmunoResearch, USA). The nucleus of cells was stained with mounting medium-DAPI. After the indirect labelling, the cells were examined with a Zeiss Laser Scanning Microscope Meta 510.

Figure 6A shows confocal images illustrating the internalization of NPs by HUVECs. DAPI stains the cell nuclei blue, LysoTracker stains the endosomes red and CD31 stains the cell membrane grey. The fluorescent labelling of the NPs is green. As can be seen from Figure 6A, the PLGA-PFCE formulation was taken up by HUVECs and accumulated substantially at the endosomes. This endosomal sequestration persisted for up to 144 hours following incubation (see T144 panels in Fig. 6A), indicating relatively little self-mediated escape or disruption of endosomal membranes by the NPs themselves. Similar results have been obtained for MNCs, as shown in Figure 6B.

### 5.2 MRI imaging

The MRI images are shown in Figure 7. Fig 7B show images of PLGA-PFCE NPs (8 mg), PFCE (750µl), PLGA-PFCE-PS-labelled HUVECs (10 x10⁶ cells) and unlabelled HUVECs (10 x 10⁶ cells) and water contained in separate eppendorfs. All eppendorfs were positioned onto a support inside a ¹⁹F 7.0 T MRI volume coil. The images acquired (shown in Fig. 7A) were coronal slices in relation to the magnet orientation. The acquisition time was 10 minutes (5 averages).

### Example 6: Cell viability and long-term labelling

### 6.1 Quantification of labelling efficiency

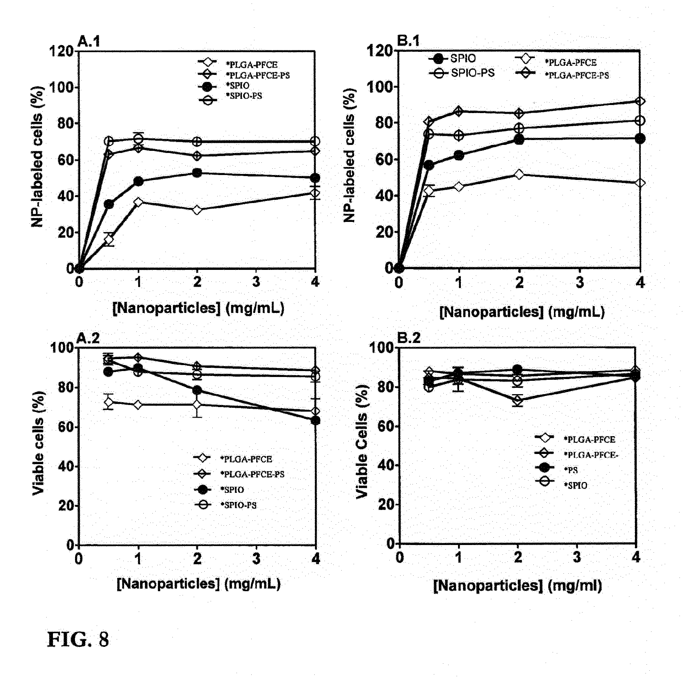

To quantify the labelling efficiency of the cells under the different conditions tested Fluorescence Activated Cell Sorting (FACS) was performed, and the results are shown in Figure 8 (A1 HUVECS; B1 MNCs).

MNCs (1×10⁶ cells) or HUVECs (0.2×10⁶ cells) were incubated for 4 hours in serum free M199 or serum free EGM-2, respectively, containing variable concentration of NPs (between 0.5 and 4 mg/ml) in a 24 well plate. After 4 hours the HUVECs were washed with PBS on the plate 3 times to remove loosely bound particles. The cells were then re-suspended in serum free EGM-2 with or without mitotracker red CMX-ROS at 50 nM for 15 minutes at 37°C in a CO₂ incubator. The cells were later trypsinised with 0.2% (w/v) trypsin solution, centrifuged at 1000 rpm for 5 minutes, and fixed with 4% paraformaldehyde for 10 minutes at room temperature. After fixation they were re-washed and then re-suspended in 500 µl of PBS, ready for FACS analysis. MNCs were transferred to a 2 ml eppendorf and centrifuged for 15 minutes at 20°C, 1300 rcfs, then the MNCs were further washed by using anti-CD45 human microbeads via Miltenyi Biotec Mini MACS system and protocol (Miltenyi Biotec, UK) so as to ensure that only MNCs were present in the final solution and no free NPs, the cells were then incubated with mitotracker (50 nM) for 15 minutes at 37 °C, washed once with PBS and fixed with 4% paraformaldehyde for 10 minutes at room temperature. After fixation, the cells were washed and resuspended in 500 µl of PBS before FACS analysis. A total of 80,000 events were recorded per measurement.

As can be seen from Figure 8, when 500 µg/ml of PLGA-PFCE-PS was incubated with cells, 65% of HUVECs and 80% of MNCs were labelled after 4 hours. According to ¹⁹F-NMR (Figure 5) this labelling corresponds to the internalization of 0.27 and 0.15 ng of PFCE per cell. This amount is enough for cells to be detected in less than 10 minutes using a ¹⁹F 7.0 T MRI volume coil. A greater amount of PS-coated PLGA-PFCE was taken up by the cells (approximately 3 times more) than uncoated ones. Interestingly, increasing the initial dose of NPs incubated with the cells did not increase the fraction of the cell population associated with fluorescence, indicative that a small percentage of cells (< 20%) were unable to taken up the NPs. Similar internalization profiles were obtained for SPIO NPs.

### 6.2 Measurement of cytotoxicity of PLGA-PFCE formulation

To evaluate the cytotoxicity of PLGA-PFCE formulation, HUVECs (in serum-free EGM2) and MNCs (M199) were exposed to variable concentrations of PLGA-PFCE NPs or SPIO NPs (as a control) for 4 hours.

At the end of the incubation, cells were washed and then cell viability was monitored by FACS using a Mitotracker CMX-ROS assay. This assay measures cell mitochondrial activity, which is directly proportional to cell viability, i.e cells positive for Mitrotracker CMX-ROS have mitochondrial activity and thus were considered as live cells. Results are shown in Figure 8 for HUVECs (see panel A) and MNCs (see panel B). Cells labelled with non-fluorescent NPs were used to define the gates during FACS acquisition. In all plots, results are average ± SD, *n*=3.

### 6.3 Effect of protamine sulphate concentration on cell viability

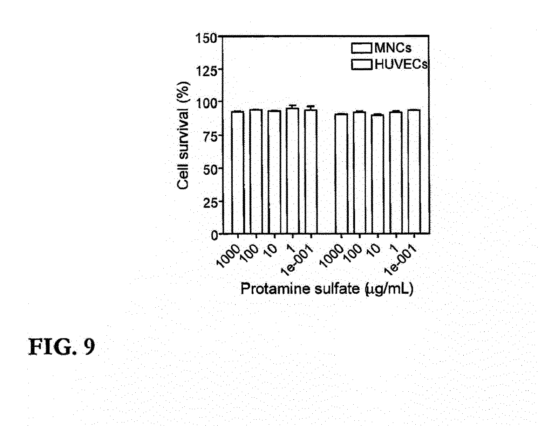

It was determined that HUVEC and MNC viability is not affected by PS at concentrations up to 1000 µg/ml (Figure 9).

### Example 7: Mechanism of NP uptake

### 7.1 Characterisation of endocytotic mechanism involved in the uptake of the NPs and long-term labelling of cells

To further elucidate the mechanism(s) of internalization, the uptake of fluorescently labelled PLGA-PFCE by HUVECs and MNCs in the presence or absence of a variety of inhibitors for known endocytotic mechanisms was characterized. Both cell types (HUVECs: 2 × 10⁵ cells per condition; MNCs: 1 × 10⁶ cells per condition) were labeled with PLGA-PFCE-PS (500 µg/ml) in the presence or absence of chemical inhibitors, at 37 °C or 4 °C, before their characterization by FACS. When inhibitors were used, the cells were pre-conditioned with the inhibitors for 1 hour prior to the addition of NPs for a further 4 hours. In the case of HUVECs, the cells were washed with PBS, trypsinised and fixed with 4% (w/v) paraformaldehyde for 10 minutes at room temperature. After fixation, the cells were washed and resuspended in 500 µl of PBS before FACS analysis. In case of MNCs, the cells were washed with PBS, separated from non-internalised NPs by MACS (using antihuman CD45 microbeads), fixed and finally characterised by FACS.

The inhibitors included cytochalasin D (Cyto D, 3 M; actin inhibitor), filipin (Fili, 3 g/ml; caveola inhibitor), and nocodazole (Noco, 10 µM; microtubule inhibitor).

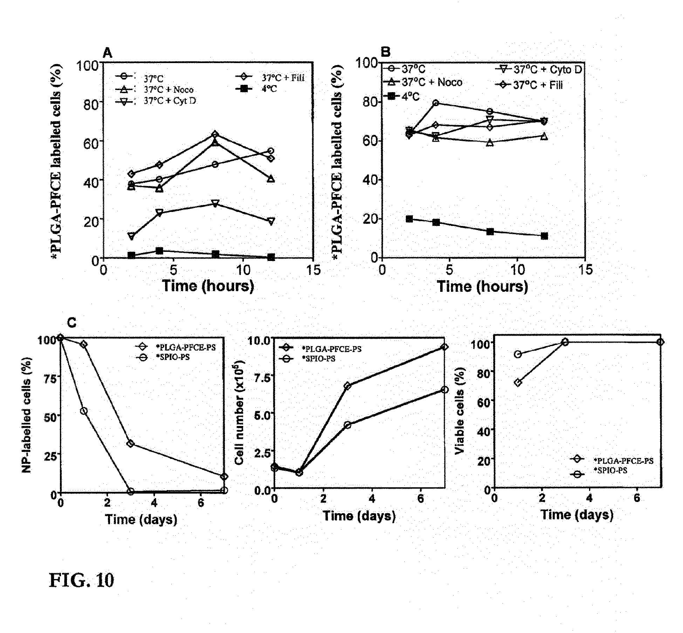

The results of the FACS analysis are shown in Figure 10A and 10B. FACS results show strong inhibition of NP uptake by HUVECs and MNCs cultured at 4 °C, demonstrating the involvement of energy-dependent endocytotic mechanisms in the uptake of the NPs. Inhibition of NP uptake by HUVECs was also observed when cells were treated with Cyt D, although significantly less than that observed for cells cultured at 4 °C. Therefore, these results indicate the involvement of actin microfilaments in the active uptake of NPs by HUVECs. In contrast, no inhibition of NP uptake was observed when MNCs were treated with Cyt D indicating differences in the mechanism of NP internalization.

### 7.2 Cell NP labelling over time

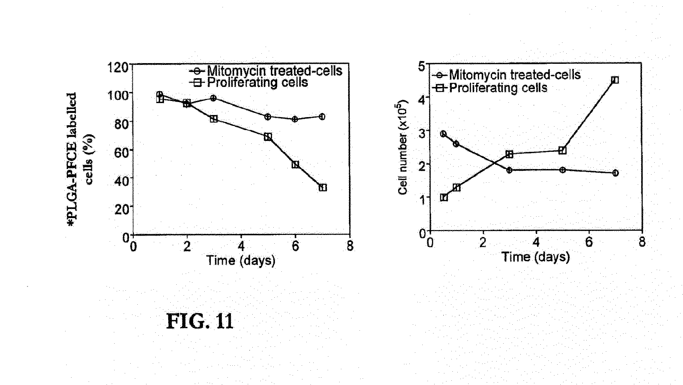

Long-term observation of PLGA-PFCE-labelled stem cells might be limited because of dilution of NPs with cell division. Therefore, cell NP labelling was quantified over time by FACS (fluorescence quantification), as shown in Figure 10C. HUVECs were labelled with PLGA-PFCE NPs for 4 hours, being the labelled cell population isolated by sorting and cultured for 7 days in EGM-2 medium. During this time, cells proliferate and have viabilities above 90% at days 3 and 7. Curiously, SPIO-labelled cells proliferate less than PLGA-PFCE-labelled cells. Importantly, the percentage of PLGA-PFCE- and SPIO-labelled cells decreased over time. Only 30% of the cells were positive for the presence of PLGA-PFCE after 4.7 cell doublings while almost none of the cells were positive for the presence of SPIO during the same number of doublings (Figure 10). The decrease in PLGA-PFCE-labelled cells is due to cell division since inhibiting the cell cycle by mitomycin, the intracellular level of NPs remains fairly constant over 7 days (Figure 11).

### Example 8: Complexation of NPs with miRNAs and cell transfection

### 8.1 Pro-survival miRNA delivery

In order to determine whether the PLGA-PFCE formulation could be used to deliver pro-survival miRNAs within cells, the following procedure was followed. Firstly, three different miRNAs were selected: miR-132 (Ambion), miR-424 (Ambion) and amiR-92a (antagomir-92a (Exiqon)), for complexation with FITC-labelled PLGA-PFCE as follows.

FITC-PLGA-PFCE NPs were weighed, sterilised under UV light for 30 minutes, and resuspended in EGM-2 serum free medium to yield a final concentration of 500 µg/ml. The suspension of NPs was then dispersed by ultrasound (2 × 10 s, BRANSON 2510), and miRNAs added (100nM and 200 nM) and allowed to complex to the NPs for 1 hour at 37 °C, with intermittent agitation. Cells were washed 2 times with pre-warmed PBS and the NP-miRNA conjugate was added to the cells for incubation at 37 °C for 4 hours. Cells were transfected keeping a ratio of 1.25 mg of NP-miRNA per million HUVEC cells.

To evaluate the potential of our approach, we performed the same experiment using a commercial transfection agent- siPORT NeoFX. In a SiPORT (Ambion) transfection procedure, SiPORT transfection agent and miRs were diluted in EGM-2 serum free medium and incubated at room temperature separately. After 10 minutes, both solutions were combined and incubated for another 10 minutes to allow the complexation of both components. The suspension was then dispensed on top of PBS-pre-washed cells and incubated for 4 hours at 37 °C, as in the PLGA procedure.

### 8.2 Confocal imaging

For transfection visualisation under the fluorescence microscope FITC-PLGA-PFCE NPs and miRIDIAN microRNA mimic transfection control with Dy547 (miR-Dy547) (Dharmacon) (100 nm) were used.

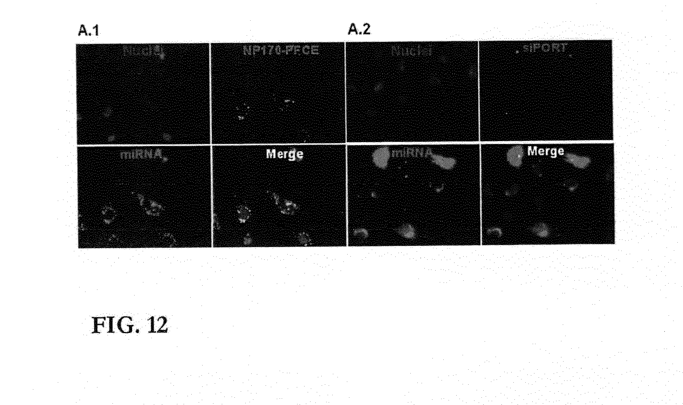

As can be seen from Figure 12, panel A, the complex miR-Dy547: FITC-PLGA-PFCE was highly taken up by cells. Over 90% of the cells were transfected with miRNA. FITC-PLGA-PFCE NPs taken up by HUVECs were in general localised in restricted areas of the cells. Most of the miRNA co-localised with the particles, while a small amount of miRNA was distributed across the cell (Figure 12, panel A). In contrast, and as can be seen from Figure 12, panel A2, the miR-Dy547 released by siPORT NeoFX was distributed across the cell cytoplasm and not in confined intracellular areas. Overall, our results show that HUVECs internalise the miRNAs and their intracellular distribution is dependent on the NP formulation.

### 8.3 Pro-survival activity

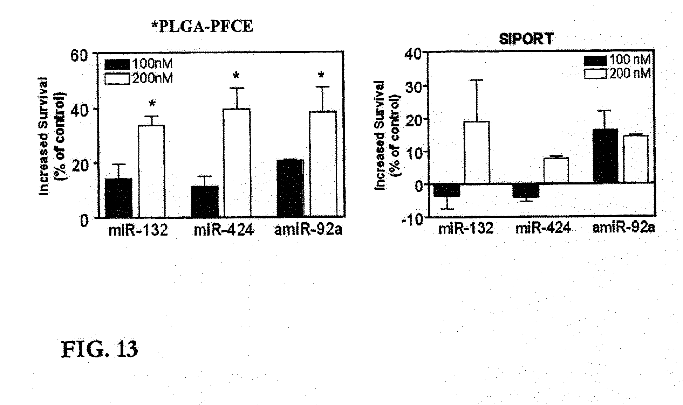

To demonstrate the pro-survival activity of different miRNAs formulations, HUVECs were incubated for 4 hours with miRNA-complexed NPs, washed, and finally cultured in ischaemic conditions (pO₂ of 0.1%; media without glucose) for 48 hours. Both miR-132, miR-424 and amiR-92a PLGA-PFCE NP formulations contribute significantly (*P*<0.05) to the survival of the cells, and this effect is dependent on the concentration of miRNA (Figure 13A). No significant differences exist between the different miRNAs tested (P>0.05). Interestingly, miRNAs delivered by siPORT transfection agent mediates cell survival only for higher concentrations than the ones observed for PLGA-PFCE (Figure 13B).

### 8.4 Survival and angiogenesis assays

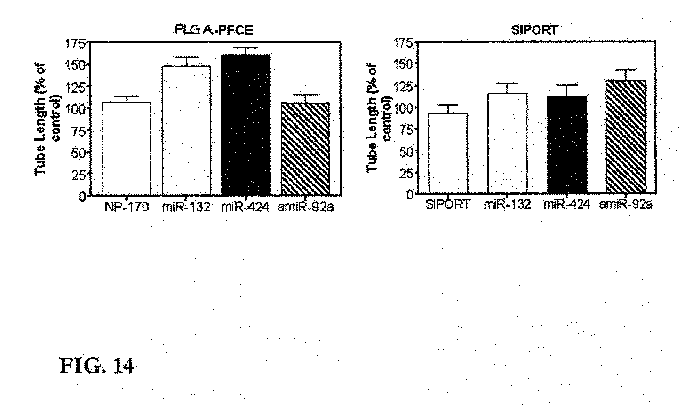

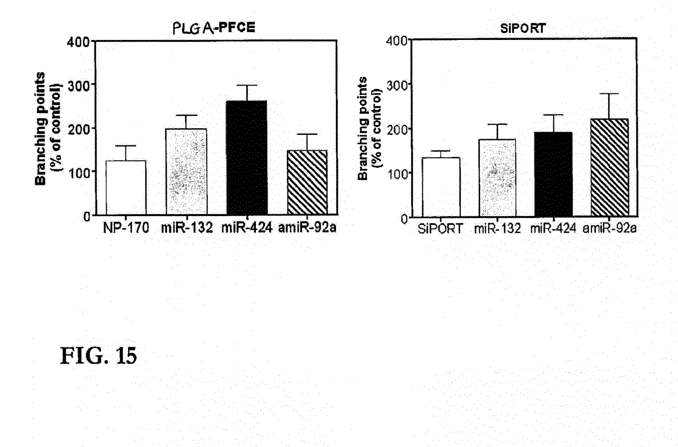

To demonstrate the pro-angiogenic effects of the miRNA-containing NP formulations, HUVECs were incubated for 4 hours with miRNA-complexed NPs, washed, and finally cultured on top of Matrigel to assess their capacity to form vascular networks. After transfection cells were washed 3 times with warm PBS and were left in complete EGM2 medium overnight to recover from transfection procedures. Next morning the cells were washed with PBS and survival challenge was done by incubating cells under oxygen (0.1%) and serum deprivation conditions for another 48 hours. Survival was assessed using the ATP-based assay Cell Titer Glow (Promega). *In vitro* angiogenesis assay was conducted using micro-angiogenesis slides (IBIDI) and Matrigel (BD) according to manufacturer instructions. After 24 hours tube length and number of branching points was measured (in a blind way) and compared between conditions. The transfection of the cells with miR-132- and miR-424-containing PLGA-PFCE NPs yielded vascular networks with higher tube length and branching points than cells transfected with amiR-92a (Figure 14, Figure 15). In contrast, the transfection of the cells with siPORT containing amiR-92a yielded vascular networks with higher tube length and branching point than cells transfected with miR-132 and miR-424.

### Statistical analysis

Throughout the examples described above, statistical analysis was performed as follows.

For analysis involving three or more groups, ANOVA was used, followed by a Student-Newman-Keuls post hoc test. For analysis of two groups, a paired t-test was used. Statistical analysis was performed using GraphPad Prism software (San Diego, CA, USA, http://www.graphpad.com/). Results were considered significant when *P*≤ 0.05.

Many modifications and variations to the described examples are possible. For example, any of the NPs described herein can be customised in terms of content (polymer, therapeutic agent, imaging agent), coating, surface charge and size. The NPs are also adaptable to any imaging method, notably MRI, fluorescence imaging and confocal microscopy.

Any of the features of any embodiment may thus be combined with any of the features of any other embodiment. Any and all combinations of the aspects and embodiments described herein are envisaged and encompassed.

## Claims

1. A nanoparticle conjugate for intracellular delivery of a therapeutic agent, wherein the conjugate comprises a nanoparticle, a cationic agent and the therapeutic agent.

2. A nanoparticle conjugate as claimed in claim 1, wherein the cationic agent is a polycationic agent.

3. A nanoparticle conjugate as claimed in claim 2, wherein the polycationic agent is selected from the group consisting of chitosan, peptides, peptide derivatives, polyethylenimine, poly(amido ethylenimine), poly(amido)amines, poly(disulphide)amines and protamine sulphate.

4. A nanoparticle conjugate as claimed in claim 3, wherein the polycationic agent is protamine sulphate.

5. A nanoparticle conjugate as claimed in any preceding claim, wherein the intracellular delivery is mediated by endosomes.

6. A nanoparticle conjugate as claimed in claim 5, wherein the nanoparticles are retained in the endolysosomal compartment.

7. A nanoparticle conjugate as claimed in any preceding claim, wherein the nanoparticle is polymeric.

8. A nanoparticle conjugate as claimed in claim 7, wherein the nanoparticle is formed from poly(lactic acid-co-glycolic acid).

9. A nanoparticle conjugate as claimed in any preceding claim, wherein the conjugate further comprises at least one imaging agent.

10. A nanoparticle conjugate as claimed in claim 9, wherein the at least one imaging agent is selected from the group consisting of perfluorocarbons and fluorescent labels.

11. A nanoparticle conjugate as claimed in claim 10, wherein the perfluorocarbon is perfluoro-1,5-crown ether.

12. A nanoparticle conjugate as claimed in any preceding claim, wherein the therapeutic agent is a protein, peptide or oligonucleotide.

13. A nanoparticle conjugate as claimed in claim 12, wherein the oligonucleotide is miRNA.
